# EUROPEAN PATENT SPECIFICATION

(11) **EP 2 523 897 B1**
(45) Date of publication and mention of the grant of the patent: **24.02.2021**
(21) Application number: 11733212.2
(22) Date of filing: 06.01.2011
(51) Int. Cl.: C10J 3/52, C10J 3/72

(54) **PRODUCING LOW METHANE SYNGAS FROM A TWO-STAGE GASIFIER**
HERSTELLUNG EINES SYNTHESEGASES MIT GERINGEM METHANANTEIL AUS EINEM ZWEISTUFIGEN VERGASER
PRODUCTION DE GAZ DE SYNTHÈSE À FAIBLE TENEUR EN MÉTHANE À PARTIR D'UN APPAREIL DE GAZÉIFICATION À DEUX ÉTAGES

(30) Priority: 05.01.2011 US 985051; 12.01.2010 US 294354 P
(43) Date of publication of application: 21.11.2012
(73) Proprietor: Lummus Technology LLC, Bloomfield, NJ 07003 (US)
(72) Inventor: JI, Shuncheng, Katy Texas 77450 (US); WILLIAMS, Chancelor L., Katy Texas 77494 (US); HERBANEK, Ron W., Richmond Texas 77406 (US)
(74) Representative: Osha Liang
(86) International application number: PCT/US2011/020340
(87) International publication number: WO 2011/087951

(56) References cited:
- WO-A1-03/022959
- WO-A2-2008/113766
- JP-A- S6 092 391
- JP-A- H06 184 562
- US-A- 4 248 604
- US-A- 4 468 376
- US-A- 4 781 731
- US-A- 5 866 091
- US-A1- 2003 116 472
- US-A1- 2006 101 715
- US-A1- 2007 051 043
- US-A1- 2008 028 680
- US-A1- 2009 038 222
- US-A1- 2009 126 276
- US-A1- 2009 198 090

## Description

### FIELD OF THE DISCLOSURE

The present invention relates to a gasification process that converts carbonaceous feedstock into desirable gaseous products such as synthesis gas. More specifically, the present disclosure relates to an improvement that allows the production of synthesis gas from a gasification reactor that has a low methane concentration. The inventive process does not require expensive treatment of the syngas to remove excess methane prior to utilization of the syngas as a feedstock for the industrial-scale production of a variety of chemicals.

### BACKGROUND

Gasification processes are widely used to convert solid or liquid feedstocks such as coal, petroleum coke and petroleum residue into synthesis gas. Synthesis gas is predominantly composed of hydrogen gas (H₂) and carbon monoxide (CO), and is utilized both as fuel for the production of electricity, as well as a feedstock for producing chemicals such as hydrogen, methanol, ammonia, synthetic/substitute natural gas or synthetic transportation oil. Synthesis gas produced via the gasification of carbonaceous material commonly contains some methane. The relative quantity of methane in the synthesis gas varies with the type of gasification system utilized, but is often observed to be higher in two-stage systems, such as ConocoPhillips E-Gas™ two-stage gasifier. Another example is the fixed-bed dry-bottom gasifier design of Lurgi GmbH (Frankfurt). In general, a significant amount of methane may be present in the syngas produced by any gasification system where the syngas leaves the reactor at a temperature of less than 1093°C (2000°F).

The two-stage gasifier configuration has the benefit of a higher energy efficiency because a portion of the sensible heat in the hot synthesis gas leaving the first stage is utilized to gasify a portion of the feedstock added to the second stage in the absence of oxygen. Pyrolysis reactions dominate within the second stage and produce not just hydrogen and carbon monoxide, but also significant amounts of methane. Consequently, synthesis gas produced from a two-stage gasification reactor generally has a higher methane content than synthesis gas from most single-stage gasifier designs. For example, the synthesis gas produced in an E-Gas™ gasifier (ConocoPhillips Co.) usually contains between 1.5 - 4% methane (dry volume). This quantity of methane is not of significant concern when the synthesis gas produced is to be utilized as fuel for gas combustion turbines that generate electricity. However, this level of methane is not desirable when the synthesis gas is to be utilized as a feedstock for the production of chemicals, since H₂ and CO are the components of synthesis gas utilized as feedstock for these chemical production processes, and in some instances, the presence of methane is detrimental to the intended chemical production process. An example of this is the production of butyraldehyde, where the process requires a feedstock synthesis gas containing less than 0.6% methane (by volume).

JP H06 184562 A discloses a process using a gasification reactor comprising (i) a thermal cracking chamber having an outlet for exiting part of the produced syngas, (ii) a slag formation chamber wherein pulverized coal and char are partially oxidized, and a (iii) slag pot, in which molten slag is cooled using cooling water, and having an outlet for exiting granulated slag and an outlet for exiting another part of the produced syngas. The syngas exiting the slag pot is combined with the syngas exiting the thermal cracking chamber. The combined syngas is introduced into a gas purification facility.

Current chemical production methods utilizing synthesis gas with a high methane content as feedstock commonly require separation of the methane from the raw synthesis gas. The resulting methane-rich purge gas is sometimes combusted as a fuel gas, or converted to additional hydrogen and carbon monoxide via steam-reforming of the methane at high temperature and pressure in the presence of a catalyst. Alternatively, the methane may be removed by other processes, such as cryogenic lean-oil absorption. However, these processes are expensive to build and operate. Accordingly, there exists a need for improved technology that allows production of raw synthesis gas with a decreased methane content by any gasification reactor wherein the synthesis gas normally leaves the reactor at a temperature less than 1093°C (2000°F). The invention described herein provides a unique process for providing a low-methane synthesis gas as a feedstock for chemical production processes without the need for expensive pre-treatment to remove methane.

### SUMMARY OF THE INVENTION

In the present invention, there is provided a method for gasifying a carbonaceous feedstock according to claim 1. The method generally comprises partially oxidizing the feedstock in a gasification reactor, thereby producing a product gas comprising H₂, CO, and a small percentage of methane. The methane content of the product gas is generally between about 0.01% and 1.5% (dry volume), but preferentially is below 0.6%, as methane content above 0.6% may interfere with utilization of the product gas as feedstock for a number of chemical production processes.

According to a first aspect, the present disclosure is directed to a process that includes the following steps: a) providing a gasification reactor that comprises at least one reaction zone having a first outlet and a quench section having a second outlet, wherein the quench section cools molten ash and removes said molten ash via the second outlet; b) partially oxidizing a first carbonaceous feedstock in the at least one reaction zone to produce a product gas comprising H₂, CO, and methane, wherein a first portion of the product gas exits the reactor via the first outlet; c) extracting a second portion of the product gas from a third outlet in the quench section of the gasification reactor, thereby producing an extraction gas stream comprising a reduced methane content relative to the first portion of the product gas of step b); d) routing the extraction gas stream via a conduit to a chemical production process, wherein the extraction gas stream serves as second carbonaceous feedstock for the production of chemicals. The percentage of methane in the extraction gas may be between about 0.01% and about 1.5% (by volume), but preferably, it is less than 0.6% (by volume).

In certain embodiments, the methane-depleted extraction gas stream is utilized as a second carbonaceous feedstock for any of a variety of chemical production processes, such as, for example, a Fischer-Tropsch process, or a process for the production of methanol, methyl acetate, urea, urea ammonium nitrate, hydrogen gas, butyraldehyde, etc.

### BRIEF DESCRIPTION OF THE DRAWINGS

Advantages of the present invention will become apparent to those skilled in the art with the benefit of the following description and upon reference to the accompanying drawing.

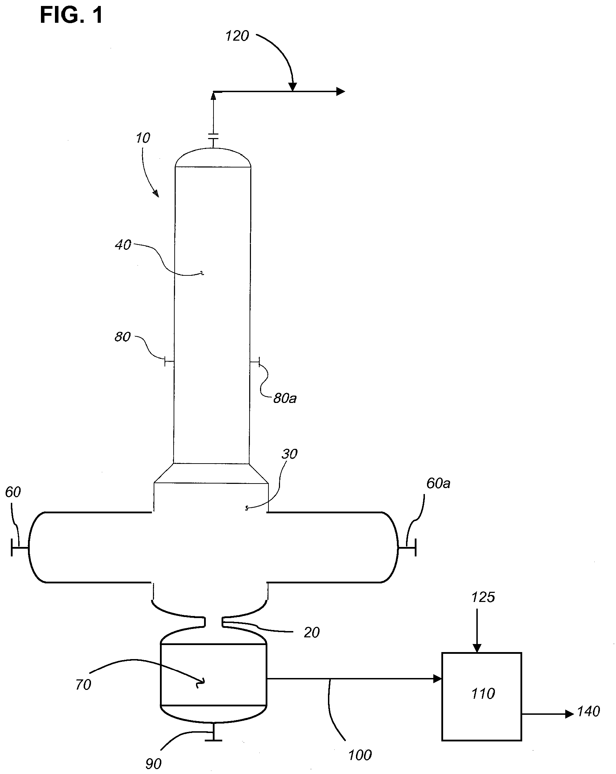

FIG. 1 is a schematic illustration in accordance with one embodiment of the current invention that depicts a two-stage gasification reactor, showing the relative positioning of the stages, as well as inlets for carbonaceous feed stock and an outlet for obtaining an extraction gas with a low methane content.

The invention is susceptible to various modifications and alternative forms, and a specific embodiment thereof is shown by way of example in the drawings. The drawings are not intended to be drawn to scale. It should be understood that the drawings and their accompanying detailed descriptions are not intended to limit the scope of the invention to the particular embodiments disclosed. Rather, the intention is to cover all modifications, equivalents and alternative embodiments falling within the spirit and scope of the present invention as defined by the appended claims.

### DETAILED DESCRIPTION

The present invention is applicable to any gasification system wherein the temperature of the synthesis gas produced is normally less than 1093°C (2000°F), and the methane content of the produced synthesis gas prevents its utilization as a feedstock for chemical production without first removing at least a portion of the methane. Such production processes may include, but are not limited to, a coal-to-liquids plant, or the production of hydrogen, ammonia, urea, methanol, or butyraldehyde.

The details of the gasification process are well-known in the art, and therefore, are described herein only in the detail required to fully disclose the present invention. In certain embodiments, the present invention builds upon the disclosures of U.S. patent applications 12/732,290, 12/640,226, 12/652,318, 12/192471 and 11/834751.

In certain embodiments of the current invention, gasification is accomplished by partial combustion of a carbonaceous feedstock with air or high purity oxygen in a gasification reactor, creating hot synthesis gas predominantly comprising hydrogen and carbon monoxide, and also some methane. The residual mineral content of the carbonaceous feedstock forms a molten slag that is continuously removed from the gasifier. The hot synthesis gas created in a first reaction zone vaporizes and provides the heat required for the gasification of additional carbonaceous feedstock introduced into a second reaction zone. The synthesis gas exiting the gasification reactor is cooled and cleaned of particulates and chemical contaminants, and is then conditioned further prior to use either as fuel for a gas turbine, or as a feedstock for the production of chemicals. The process of the current invention relates to the use of the synthesis gas as feedstock for the production of chemicals.

The present invention provides a two-stage gasification reactor that comprises first and second reaction zones. FIG. 1 depicts a two-stage gasification reactor according to the E-Gas™ gasification reactor configuration owned by ConocoPhillips Co. The first reaction zone comprises a reactor lower-section 30, while the second reaction zone comprises a reactor upper-section 40. In FIG. 1, the unfired reactor upper-section 40 of the reactor 10 is directly attached to the top of the fired reactor lower-section 30 of the reactor 10 so that the hot reaction products of the first reaction zone are conveyed directly from the reactor lower-section 30 to the second reaction zone of the reactor upper-section 40, thereby minimizing heat loss.

Further referring to FIG. 1, the gasification process begins within the first reaction zone (or reactor lower-section 30), when a carbonaceous feedstock is mixed with a gas stream comprising an oxygen-containing gas and/or steam and a rapid exothermic reaction takes place in which the carbonaceous feedstock is converted into a first mixture product comprising steam, hydrogen, carbon monoxide, carbon dioxide, methane, and entrained particulates such as ash. Ash is comprised of the non-combustible mineral content of the carbonaceous feedstock. The temperature of the first reaction zone 30 is maintained higher than the ash melting point, which allows the ash to melt and agglomerate to form a viscous liquid known as slag. The slag falls to the bottom of the reactor lower-section 30 and flows through a taphole 20 and into a quench chamber 70, whereupon it is water-quenched and directed via an outlet 90 to a slag processing system (not shown) for final disposal.

The primary combustion reaction occurring in the first reaction zone is *C*+*½O₂→CO*, which is highly exothermic. It raises the temperature in the first reaction zone to between (1093°C (2000°F) and 1926°C (3500°F). The heat produced in the first reaction zone is carried upward with the gas stream, thereby providing heat for pyrolysis reactions that occur in the unfired second reaction zone, including vaporization of the feed water, the carbon-steam reaction and the water-gas reaction between CO and H₂O. The carbon-steam reaction forms CO and H₂, thus, increasing the yield of these usable gases.

In the embodiment shown in FIG. 1, a pulverized solid stream of carbonaceous feedstock is injected into the second reaction zone (or upper-section 40) through feeding device 80 and/or 80a. In certain alternative embodiments (not depicted), alternative feeding devices, such as, but not limited to, slurry feeding systems, can be utilized to add feedstock to the gasification reactor.

The physical conditions of the reaction in the second reaction zone (or reactor upper-section 40) are controlled to assure rapid gasification and heating of the feedstock above its range of plasticity. Once dispersed into the reactor upper-section, the feedstock comes into contact with the hot first mixture product rising from the first reaction zone (or reactor lower-section 30). The carbonaceous feedstock is dried as the water in the slurry turns to steam, and a portion of the feedstock is gasified via pyrolysis reactions such as the carbon steam reaction (*C*+*H₂O→CO*+*H₂*) to produce hydrogen and carbon monoxide.

Further referring to FIG. 1, the raw gas stream exiting the gasification reactor via a conduit 120 may comprise one or more of the following: carbon monoxide (CO), carbon dioxide (CO₂), hydrogen (H₂), water (H₂O), methane (CH₄) and other light hydrocarbons, and nitrogen (N₂). Additionally, the raw gas stream can comprise one or more undesirable components (i.e., contaminants) that should be removed prior to utilizing the raw gas stream for the production of chemicals. Sulfur compounds, such as, for example, hydrogen sulfide (H2S), carbonyl sulfide (COS), carbon disulfide (CS₂), and even organosulfur compounds such as mercaptans and various thiophenic compounds are a few examples of common contaminants found in the raw gas stream. Other examples of contaminants typically present in the raw gas stream can include, but are not limited to ammonia (NH₃), hydrochloric acid (HCl), and hydrogen cyanide (HCN). The high-temperature raw synthesis gas is cooled, and both particulates and acid gases are removed via methods that are commonly known to those skilled in the art and that are outside the scope of this document (not depicted). The resulting cleaned syngas can then be utilized as fuel to power a gas turbine, but for some chemical applications, excess methane must be removed prior to use of the syngas as feedstock any of a variety of chemical production processes.

Table 1, below, summarizes the composition of the raw synthesis gas stream according to certain embodiments of the present invention, wherein syngas is produced by a two-stage slurry-fed gasifier of the E-Gas™ configuration.

**Table 1:**

| **Component** | **Component in Raw Gas Stream (based on total stream volume)** | | |
|---|---|---|---|
| | **Broad Range** | **Intermediate Range** | **Narrow Range** |
| H₂ | 8 - 50 vol% | 10 - 40 vol % | 15-35 vol % |
| CO | 10 - 75 vol % | 15-60 vol % | 25 - 50 vol % |
| CO₂ | 1 - 40 vol % | 5-30 vol % | 7 - 20 vol % |
| H₂O | 4 - 40 vol % | 8 - 30 vol % | 10 - 25 vol % |
| H₂S | 0.001 - 5 vol % | 0.1 - 2.5 vol % | 0.5 - 2 vol % |
| CH₄ | 0.05 - 10 vol % | 0.1 to 7.5 vol % | 0.5 to 5.0 vol % |
| COS | 100 - 5,000 ppmv | 200 - 2,500 ppmv | 350 - 1,500 ppmv |
| HCl | 50 - 2,000 ppmv | 100 - 1,500 ppmv | 250 - 1,000 ppmv |
| NH₃ | 50 - 2,000 ppmv | 100 - 1,500 ppmv | 250 - 1,000 ppmv |
| Other (total) | < 2.5 vol % | < 2.0 vol % | < 1 vol % |

Table 1 shows that the raw syngas stream produced from the second reaction zone (or upper section 40) of the gasifier may contain a significant amount of methane (up to 10% by volume). Methane is not a useful feedstock component for many chemical synthesis processes that utilize the H₂ and CO components of synthesis gas as a starting material, and in some instances the presence of methane impedes the chemical synthesis.

Referring again to FIG. 1, established methods withdraw an extraction gas 100 from the quench chamber 70 in order to create a negative pressure that encourages the flow of molten ash from the gasification reactor through the taphole 20 and into the quench chamber 70. The negative pressure created by withdrawing the extraction gas also prevents plugging of the taphole by molten ash. According to established methodology, the extraction gas 100 is combined with the raw synthesis gas 120 obtained from the second reaction zone (upper section 40) upstream from the particulate removal system (not depicted).

However, the process of the present invention provides a process for obtaining syngas from a gasification reactor that has a methane content that is far lower than the methane content of the syngas produced in the second reaction zone (upper section 40). In certain embodiments, the methane content of the low-methane syngas obtained may be lower than about 1.5% (dry volume). Preferably, the methane content of the low-methane syngas obtained may be lower than about 0.6% (dry volume). Most preferably, the methane content of the low-methane syngas obtained may be lower than about 0.25% (dry volume). In the present invention, the extraction gas 100 is directed via a conduit to a wash drum 110. The wash drum contains an inlet for water 125, and washes the extraction gas to remove particulates. Methods and apparatus associated with such wash drums are commonly known to those skilled in the art. A particulate-free synthesis gas is produced 140 and can be utilized as feedstock for a variety of chemical production processes without further pretreatment.

### EXAMPLE 1

According to the process of the current invention, an extraction gas 100 is obtained from an outlet in the quench chamber 70. This extraction gas comprises a synthesis gas that is much lower in methane content than the syngas emitted from the second reaction zone (upper stage 40) of the gasifier. An analysis was performed to determine the composition of the synthesis gases emitted from a two-stage slurry-fed E-Gas™ gasifier. Gas samples were taken from both the top of the second reaction zone 120, as well as an extraction gas outlet 100 in the quench chamber 70. The chemical composition of the two samples was analyzed, and the relative quantities of various gaseous components within each sample are presented in Table 2.

**Table 2:**

| **Component** | **Syngas From Second Reaction Zone (mol fraction)** | **Extraction Gas (mol fraction)** |
|---|---|---|
| N₂ | 1.5% | 1.7% |
| Ar | 0.8% | 1.0% |
| H₂ | 35.3% | 32.3% |
| CH₄ | 2.7% | 0.1% |
| CO | 42.8% | 53.1% |
| CO₂ | 16.1% | 11.6% |
| H₂S | 0.4% | 0.2% |
| NH₃ | 0.4% | 0.0% |
| Total | 100.0% | 100.0% |

Table 2 shows that the methane content of the synthesis gas obtained from the top of the second reaction zone had a much higher methane content (2.7%) than the extraction gas obtained from the quench chamber (0.1%) The extraction gas also had a reduced hydrogen sulfide and ammonia content. The extraction gas is then subjected to a simple cleaning procedure in a conventional wash drum to provide a particulate-free synthesis gas that is suitable for use as a feedstock for a variety of chemical production processes.

The process of the current invention is applicable to a variety of different chemical production processes (as described) that utilize the components of synthesis gas as a feedstock. In one embodiment of the of the present invention, petroleum coke produced by a refinery is utilized as a carbonaceous feedstock for a two-stage, slurry-fed gasification reactor. The syngas produced by gasification of the feedstock may serve as a source of hydrogen gas for certain refinery processes (such as hydrotreating, etc.) as well as a feedstock for any petrochemical unit that requires a low-methane syngas as its feedstock. One example is an "Oxo" Process, which involves the hydroformylation of propylene to butyraldehyde, where the main reaction is as follows:

CH₃CH=CH₂ + CO + H₂ → CH₃-CH₂-CH₂-CHO

This chemical synthesis process requires that the methane content of the syngas feedstock is less than 0.6% (dry volume), a requirement that is readily met by the reduced-methane extraction gas of the present invention (as shown in Table 2) without requiring expensive pre-treatment of the syngas to remove methane.

### DEFINITIONS

For the purposes of this disclosure, the term "syngas" is synonymous with synthesis gas or synthetic gas, the term "gas" is synonymous with methane, natural gas, as well as gasoline or any other liquid hydrocarbon fuel.

For the purposes of this disclosure, use of the terms "low methane syngas" or "low-methane synthesis gas" refers to a syngas that has a methane content of 1.5% (dry volume) or less.

All gas composition percentages are expressed as dry volume unless indicated otherwise.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed and certain features of the invention may be utilized independently, all as would be apparent to one skilled in the art after having the benefit of this description of the invention. Changes may be made in the elements described herein without departing from the scope of the following claims.

## Claims

1. A process comprising the following steps:
a) providing a gasification reactor (10) that comprises:
at least one reaction zone (30, 40) comprising:
a first reaction zone (30), and
a second reaction zone (40) including a first outlet; and
a quench section (70) including a second outlet (90), wherein the quench section (70) is configured to cool molten ash and remove said molten ash via the second outlet (90), and wherein the first reaction zone (30) is placed intermediate the second reaction zone (40) and the quench section (70);
b) feeding a carbonaceous feedstock to the first reaction zone (30) and feeding additional carbonaceous feedstock in form of a slurry to the second reaction zone (40);
c) partially oxidizing the carbonaceous feedstock in the at least one reaction zone (30, 40) to produce a product gas comprising H₂, CO, and methane exiting the reactor via the first outlet and molten ash;
d) extracting an extraction gas stream (100) from a third outlet in the quench section (70) of the gasification reactor (10), the extraction gas stream (100) having a reduced methane content relative to the product gas of step c), namely between about 0.01% and about 1.5% methane based on dry volume;
e) cooling the molten ash in the quench section (70) and removing said molten ash via the second outlet (90); and
f) rerouting the extraction gas stream (100) to serve as a feedstock for a chemical production process that comprises a Fischer-Tropsch process, or a process for the production of methanol, ammonia, methyl acetate, urea, urea ammonium nitrate, butyraldehyde or hydrogen gas,
wherein:
the first reaction zone (30) is fired and the second reaction zone (40) is unfired,
a partial oxidation of the carbonaceous feedstock is the predominant reaction occurring within the first reaction zone (30), while pyrolysis of the carbonaceous feedstock is the predominant reaction occurring within the second reaction zone (40) via the heat produced in the first reaction zone (30), and
the partial oxidation of the carbonaceous feedstock raises the temperature of the first reaction zone (30) to between 1093°C (2000°F) and 1926°C (3500°F).

2. The process of claim 1, wherein step d) comprises routing the extraction gas stream (100) to a wash drum (110), wherein said wash drum (110) utilizes water to remove a majority of particulates from the extraction gas stream (100), thereby creating a cleaned extraction gas stream (100); and routing the cleaned extraction gas stream (100) to serve as the feedstock for the chemical production process.

3. The process of claim 1 or 2, wherein the extraction gas stream (100) contains less than about 0.6% methane based on dry volume.

4. The process of claim 1 or 2, wherein the extraction gas stream (100) contains less than about 0.25% methane based on dry volume.

5. The process of claim 1, wherein the extraction gas stream (100) is routed to a wash drum (110), wherein said wash drum (110) utilizes water to remove particulates from the gas.

6. The process of claim 4 when dependent from claim 2, wherein the extraction gas stream (100) is produced from the third outlet in the quench section (70) immediately below the first reaction zone (30).

7. The process of claim 1 or 2, wherein the methane content of the product gas of part b) is between about 0.5% and 10% based on dry volume.

## Patentansprüche

1. Verfahren mit den folgenden Schritten:
a) Bereitstellen eines Vergasungsreaktors (10), welcher aufweist:
mindestens eine Reaktionszone (30, 40) mit:
einer ersten Reaktionszone (30), und
einer zweiten Reaktionszone (40), die einen ersten Auslass aufweist; und
einen Abschreckabschnitt (70) mit einem zweiten Auslass (90), wobei der Abschreckabschnitt (70) dazu ausgebildet ist, geschmolzene Asche abzukühlen und die geschmolzene Asche über den zweiten Auslass (90) zu entfernen, und wobei die erste Reaktionszone (30) zwischen der zweiten Reaktionszone (40) und dem Abschreckbereich (70) angeordnet ist,
b) Zuführen eines kohlenstoffhaltigen Beschickungsmaterials zu der ersten Reaktionszone (30) und Zuführen von zusätzlichem kohlenstoffhaltigem Beschickungsmaterial in Form eines Schlamms zu der zweiten Reaktionszone (40);
c) teilweises Oxidieren des kohlenstoffhaltigen Beschickungsmaterials in der mindestens einen Reaktionszone (30, 40), um ein Produktgas, das H₂, CO und Methan, das den Reaktor über den ersten Auslass verlässt, enthält und geschmolzene Asche zu erzeugen;
d) Extrahieren eines Extraktionsgasstroms (100) aus einem dritten Auslass in dem Abschreckabschnitt (70) des Vergasungsreaktors (10), wobei der Extraktionsgasstrom (100) einen reduzierten Methangehalt in Bezug auf das Produktgas von Schritt c) aufweist, nämlich zwischen ungefähr 0,01% und ungefähr 1,5 % Methan basierend auf dem Trockenvolumen;
e) Kühlen der geschmolzenen Asche in dem Abschreckabschnitt (70) und Entfernen der geschmolzenen Asche über den zweiten Auslass (90); und
f) Leiten des Extraktionsgasstroms (100), so dass dieser als ein Beschickungsmaterial für einen chemischen Produktionsprozess dient, der einen Fischer-Tropsch-Prozess oder einen Prozess für die Produktion von Methanol, Ammoniak, Methylacetat, Harnstoff, Harnstoff-Ammoniumnitrat, Butylaldehyd oder Wasserstoffgas aufweist,
wobei:
die erste Reaktionszone (30) befeuert ist und die zweite Reaktionszone (40) nicht befeuert ist,
eine teilweise Oxidation des kohlenstoffhaltigen Beschickungsmaterials die vorherrschende Rektion ist, die in der ersten Reaktionszone (30) auftritt, während die Pyrolyse des kohlenstoffhaltigen Beschickungsmaterial die vorherrschende Reaktion ist, die in der zweiten Reaktionszone (40) mittels der in der ersten Reaktionszone (30) erzeugten Wärme auftritt, und
die teilweise Oxidation des kohlenstoffhaltigen Beschickungsmaterials die Temperatur der ersten Reaktionszone (30) auf zwischen 1093°C (2000°F) und 1926°C (3500°F) erhöht.

2. Verfahren nach Anspruch 1, bei welchem der Schritt d) aufweist: das Leiten des Extraktionsgasstroms (100) zu einer Waschtrommel (110), wobei die Waschtrommel (110) Wasser verwendet, um den Großteil der Partikel aus dem Extraktionsgasstrom (100) zu entfernen, wodurch ein gereinigter Extraktionsgasstrom (100) erzeugt wird; und das Leiten des gereinigten Extraktionsgasstroms (100) derart, dass er als das Beschickungsmaterial für den chemischen Produktionsprozess dient.

3. Verfahren nach Anspruch 1 oder 2, bei welchem der Extraktionsgasstrom (100) weniger als ungefähr 0,6% Methan basierend auf dem Trockenvolumen enthält.

4. Verfahren nach Anspruch 1 oder 2, bei welchem der Extraktionsgasstrom (100) weniger als ungefähr 0,25% Methan basierend auf dem Trockenvolumen enthält.

5. Verfahren nach Anspruch 1, bei welchem der Extraktionsgasstrom (100) zu einer Waschtrommel (110) geleitet wird, wobei die Waschtrommel (110) Wasser verwendet, um Partikel aus dem Gas zu entfernen.

6. Verfahren nach Anspruch 4, wenn auf Anspruch 2 rückbezogen, bei welchem der Extraktionsgasstrom (100) aus dem dritten Auslass in dem Abschreckabschnitt (70) unmittelbar unter der ersten Reaktionszone (30) produziert wird.

7. Verfahren nach Anspruch 1 oder 2, bei welchem der Methangehalt des Produktgases des Teils b) zwischen ungefähr 0,5% und 10% basierend auf dem Trockenvolumen beträgt.

## Revendications

1. Procédé comprenant les étapes suivantes :
a) la fourniture d'un réacteur de gazéification (10) qui comprend :
au moins une zone de réaction (30, 40) comprenant :
une première zone de réaction (30), et
une seconde zone de réaction (40) incluant une première sortie ; et
une section de trempe (70) incluant une deuxième sortie (90), dans laquelle la section de trempe (70) est configurée pour refroidir les cendres fondues et pour retirer lesdites cendres fondues par la deuxième sortie (90), et dans laquelle la première zone de réaction (30) est placée entre la seconde zone de réaction (40) et la section de trempe (70) ;
b) l'alimentation de la première zone de réaction (30) en charge carbonée et l'alimentation de la seconde zone de réaction (40) en charge carbonée additionnelle sous forme de suspension ;
c) l'oxydation partielle de la charge carbonée dans l'au moins une zone de réaction (30, 40) pour produire un produit gazeux comprenant H₂, CO et du méthane sortant du réacteur par la première sortie et des cendres fondues ;
d) l'extraction d'un flux de gaz d'extraction (100) à partir d'une troisième sortie dans la section de trempe (70) du réacteur de gazéification (10), le flux de gaz d'extraction (100) ayant une teneur en méthane réduite par rapport au produit gazeux de l'étape c), à savoir entre environ 0,01% et environ 1,5% de méthane sur la base du volume sec ;
e) le refroidissement des cendres fondues dans la section de trempe (70) et l'évacuation desdites cendres fondues par la deuxième sortie (90) ; et
f) l'acheminement du flux de gaz d'extraction (100) pour servir de charge d'alimentation pour un procédé de production chimique qui comprend un procédé Fischer-Tropsch, ou un procédé de production de méthanol, d'ammoniac, d'acétate de méthyle, d'urée, d'urée-nitrate d'ammonium, de butyraldéhyde ou d'hydrogène gazeux,
dans lequel :
la première zone de réaction (30) est brûlée et la seconde zone de réaction (40) n'est pas brûlée,
une oxydation partielle de la charge carbonée est la réaction prédominante se produisant dans la première zone de réaction (30), tandis que la pyrolyse de la charge carbonée est la réaction prédominante se produisant dans la seconde zone de réaction (40) par l'intermédiaire de la chaleur produite dans la première zone de réaction (30), et
l'oxydation partielle de la charge carbonée fait monter la température de la première zone de réaction (30) à une température comprise entre 1093°C (2000°F) et 1926°C (3500°F).

2. Procédé selon la revendication 1, dans lequel l'étape d) comprend l'acheminement du flux de gaz d'extraction (100) vers un tambour de lavage (110), dans lequel ledit tambour de lavage (110) utilise de l'eau pour éliminer une majorité de particules du flux de gaz d'extraction (100), créant ainsi un flux de gaz d'extraction nettoyé (100) ; et l'acheminement du flux de gaz d'extraction nettoyé (100) pour servir de charge au procédé de production chimique.

3. Procédé selon la revendication 1 ou la revendication 2, dans lequel le flux de gaz d'extraction (100) contient moins d'environ 0,6 % de méthane sur la base du volume sec.

4. Procédé selon la revendication 1 ou la revendication 2, dans lequel le flux de gaz d'extraction (100) contient moins d'environ 0,25% de méthane sur la base du volume sec.

5. Procédé selon la revendication 1, dans lequel le flux de gaz d'extraction (100) est acheminé vers un tambour de lavage (110), dans lequel ledit tambour de lavage (110) utilise de l'eau pour éliminer les particules du gaz.

6. Procédé selon la revendication 4 lorsqu'elle dépend de la revendication 2, dans lequel le flux de gaz d'extraction (100) est produit à partir de la troisième sortie de la section de trempe (70) immédiatement en dessous de la première zone de réaction (30).

7. Procédé selon la revendication 1 ou la revendication 2, dans lequel la teneur en méthane du produit gazeux de la partie b) est comprise entre environ 0,5 % et 10 % sur la base du volume sec.
